# EUROPEAN PATENT APPLICATION

(11) **EP 2 058 657 A1**
(43) Date of publication of application: **13.05.2009**
(21) Application number: 07021240.2
(22) Date of filing: 31.10.2007
(51) Int. Cl.: G01N 33/50, G01N 33/543

(54) **Method for determining bioactivity of molecules**

(71) Applicant: Bayer Schering Pharma Aktiengesellschaft, 13353 Berlin (DE)
(72) Inventor: Barbin, Karin, Dr., 13086 Berlin (DE); Tiemann, Frank, Dr., 42111 Wuppertal (DE); Gerbling, Klaus-Peter, Dr., 13469 Berlin (DE)

(57) **Abstract**

The present invention relates to a method for determining activity of a molecule, wherein the molecule comprises at least two active parts, and wherein the first part is capable of modulating the activity of cells, and the second part can specifically bind to a target, and kits useful for performing the methods.

## Description

The present invention relates to a method for determining activity of a molecule, wherein the molecule comprises at least two active parts, and wherein the first part is capable of modulating the activity of cells, and the second part can specifically bind to a target, and kits useful for performing the methods.

Since 1997, when the Food and Drug Administration approved the chimeric CD20 antibody Rituximab (Rituxan; Genentech Inc, South San Francisco, CA, and Idec Pharmaceuticals, San Diego, CA) for the treatment of B-cell non-Hodgkin's lymphoma, antibody therapy has gained great importance in the treatment of human diseases (Grillo-Lopez AJ et al. 2002. Semin Oncol. 29, 105-112). Besides unconjugated antibodies also antibodies and fragments thereof conjugated to drugs, toxins, radionuclides or cytokines have been designed for targeted delivery of active components (Glennie M et al. 2000. Immunol Today 21 (8), 403-410, Presta L. 2003. Curr Opin Struct Biol. 13(4), 519-525). In contrast to radioactive components, toxins and cytotoxic drugs, that aim at destroying the target cell, cytokines fused to antibodies or antibody fragments may also act by activating the host immune system. Physiological cytokines reach high concentrations only in the proximity of the producing cells, where they can exert their function, however, systematically administered they can be associated with severe adverse reactions. To avoid these side-effects of cytokines and simultaneously take advantage of their biological benefits, antibodies have been used as payloads for targeted cytokine delivery. Different cytokines including interleukin 2 (IL-2) (Carnemolla B et al. 2002. Blood 99(5), 1659-1665; Gillies SD et al. 1992. PNAS 89(4), 1428-1432; Heuser C et al. 2004. Int J Cancer 110(3), 386-394), IL-12 (Gillies SD et al. 1998. J Immunol 160, 6195-6203; Gafner V et al. 2006. Int J Cancer 119(9), 2205-2212, Lo KM et al. 2007. Cancer Immunol Immunother 56, 447-457), IL-15 (Kaspar M et al. 2007. Cancer Res 67(10), 4940-4948), granulocyte macrophage colony stimulating factor (GM-CSF) (Dela Cruz JS et al. 2000. J Immunol 165(9), 5112-5121; Kaspar M et al. 2007. Cancer Res 67(10), 4940-4948) and tumor necrosis factor-α (TNFα) (Borsi L et al. 2003. Blood 102(13), 4384-4392; Halin C et al. 2003. Cancer Res 63(12), 3203-3210; Liu Y et al. 2004. Int J Cancer 108(4), 549-557) have been fused to antibodies or antibody fragments, and the resulting proteins have been evaluated in preclinical and in part also clinical studies. So far, the vast majority of studies have been performed with antibody-IL-2 fusion proteins.

IL-2, which was discovered 1976 as a growth factor for bone marrow-derived T lymphocytes (Morgan DA et al. 1976. Science 193, 1007-1008), is a monomeric, secreted glycoprotein with a molecular weight of approximately 15kDa. It is a potent immune-stimulatory protein exerting its effects on many cell types, including T-, B- and NK cells. (Gaffen SL et al. 2004. Cytokine 28(3), 109-123).

The targeted delivery of IL-2 has been evaluated in vitro and in vivo using several target structures expressed by tumor cells or cells of the tumor environment. The first antibody-IL2 fusion was made with ch14.18, a chimeric antibody recognizing the disialoganglioside GD2 that is expressed on tumors of neuroectodermal origin, including melanoma, neuroblastoma and certain sarcomas (Cheung NK et al. 1985. Canc Res 45(6), 2642-2649; Mujoo K et al. 1987. Canc Res 47, 1098-1104). This immunocytokine retained antigen binding capacity and IL-2 bioactivity (Gillies SD et al. 1992. PNAS 89(4), 1428-1432), and demonstrated to be effective in murine melanoma and neuroblastoma models (Becker JC et al. 1996. PNAS 93(7), 2702-2707; Pancook JD et al. 1996. Canc Immunol Immunother 42(2), 88-92; Sabzevari H et al. 1994. PNAS 91 (20), 9626-9630; Becker JC et al. 1996. J Exp Med 183 (5), 2361-2366; Lode HN et al. 1997. J Natl Cancer Inst 89(2), 1586-1594). The humanized version of this GD2 antibody hu14.18 was tested in Phase I clinical trials in adult melanoma patients as well as in pediatric patients with refractory or recurrent neuroblastoma and melanoma (King DM et al. 2004. J Clin Oncol 22(22), 4463-4473, Osenga KL et al. 2006. Clin Canc Res 12(6), 1750-1759). Although the trials were only designated to evaluate safety, toxicity and maximum tolerated dose, the clinical outcome of the patients was monitored, showing stable disease in 58% of adult melanoma patients after one course of treatment and 24% after two courses of treatment (completed by 52% of patients) (King DM et al. 2004. J Clin Oncol 22(22), 4463-4473). In the study with pediatric patients 54% had stable disease for two or more courses of immunocytokine therapy (Osenga KL et al. 2006. Clin Canc Res 12(6), 1750-1759).

Other IL-2 immunocytokines that have been evaluated in preclinical and partially in clinical studies targeted several antigens expressed on the surface of tumor cells including EpCAM (Ko YJ et al. 2004 J Immunother. 27(3), 232-239; Schanzer JM et al. 2006. Cancer Immun 6, 4), CD30 (Hombach A et al. 2005, Int J Cancer 115(2), 241-247; Heuser C et al. 2004. Int J Cancer 110(3), 386-394) and Her2/neu (Lustgarten J et al. 1999. J Immunol 162, 359-365).

Besides antibody-cytokine fusion proteins directed against target structures expressed on tumor cells, another interesting strategy is to deliver cytokines to the tumor environment. A promising candidate as fusion partner is the single-chain Fv (scFv) L19, specific for the extradomain B (ED-B), which is part of the fibronectin B isoform (ED-B FN) (Pini A et al. 1998. J Biol Chem 273(34), 21769-21776) and a marker of neoangiogenesis and tissue remodelling. ED-B FN accumulates around neovascular structures in aggressive tumors and other tissues undergoing angiogenesis, such as neoplasia, some ocular structures in pathological conditions, and fetal tissues, but is otherwise undetectable in normal adult tissues with exception of the female reproductive system (Castellani P et al. 1994. Int J Cancer 59(5), 612-618; Halin C et al. 2001. News Physiol Sci 16, 191-194; Kaczmarek J et al. 1994, Int J Cancer 59(1), 11-16).

The scFv L19 was isolated from Phage Display libraries using affinity maturation procedures, which resulted in a high affinity antibody fragment (K_{d}=54pM) (Pini A et al. 1998. J Biol Chem 273(34), 21769-21776). The selective tumor targeting properties of L19 have been demonstrated in tumor-bearing mice (Viti F et al. 1999. Cancer Res 59(2), 347-352, Tarli L et al. 1999. Blood 94(1), 192-198; Demartis S et al. 2001. Eur J Nucl. Med 28(4), 534-539) and in rabbit models of ocular angiogenesis (Birchler M et al. 1999. Nat Biotechnol 17(10), 984-988). A dimeric form of the L19 scFv labelled with ¹²³I selectively localized in tumor lesions of patients with aggressive types of lung cancer and colorectal cancer (Santimaria M et al. 2003. Clin Cancer Research 9(2), 571-579).

Due to its excellent tumor targeting properties, L19 has been fused to a plethora of active components, including cytokines as IL-2 (Carnemolla B et al. 2002. Blood 99(5), 1659-1665), IL-12 (Halin C et al. 2002. Nat Biotechnol 20(3), 264-269; Halin C et al. 2003. Cancer Res 63(12), 3202-3210), IL-15 (Kaspar M et al. 2007. Cancer Res 67(10), 4940-4948), TNF-α (Balza E et al. 2006. Clin Cancer Res 12(8), 2575-2582; Borsi L et al. 2003. Blood 102(13), 4384-4392), interferon γ (IFN-γ) (Ebbinghaus C et al. 2005. Int J Cancer 116(2), 304-313) and vascular endothelial growth factor (VEGF) (Halin C et al. 2002. Int J Cancer 102(2), 109-116). A promising candidate is L19-IL2, a recombinant immunocytokine comprising the L19 scFv and human IL-2, which is currently being clinically investigated.

To assure high and consistent quality of clinical grade material a set of validated methods is used to evaluate the physicochemical and biological properties of the protein. High demands are made on these methods with regard to assay performance in order to obtain valid results. Bioassays are assays in which concentrations of biologically active factors are quantitated by the biological effect these factors have in sensitive cells, tissues or organisms. Due to the use of cells, tissues or organisms for determining activity of the proteins, bioassays often are subject to higher assay variability. Therefore, particularly for cell-based or tissue-based bioassays, an intelligent experimental setup is necessary to produce reliable results with low error margins.

Methods for bioactivity testing of immunocytokines depend on the respective fusion partners, namely the antibody or antibody fragment and the cytokine portion. Whereas the functional activity of the antibody or antibody derivative is determined by testing specific binding to the respective antigen, the biological activity of a cytokine can be determined by the biological effect the cytokine exerts on a given cell system, including stimulation or inhibition of cell proliferation, cytotoxicity/ apoptosis, antiviral activity, chemotactic activity, differentiation, and up-regulation of expression of intracellular, secreted, and surface membrane proteins (Foster JR. 2001. Int J Exp Pathol 82(3), 171-192, Meager A. 2006. Methods 38(4), 237-252). These assays include the use of primary cells as well as established cell lines that proliferate in dependency of the given cytokine or that respond in a particular way to the cytokine.

For IL-2 antibody fusion proteins the biological activity is commonly determined with a cell-based assay, in which the proliferation of IL-2 responsive cells is analyzed. The murine cytotoxic T cell line CTLL-2 is an IL-2 dependent cell line that often is used for such assays, but also other cell lines including HT-2 (murine T-helper cell-derived), Kit-225 (human, T-cell chronic lymphocytic leukaemia-derived), TF1β (human myelomonocytic cell line transfected with human IL-2 receptor β chain gene; Farner NL et al. Blood. 1995. 86(12), 4568-4578) have been used for this purpose. IL-2 activity is determined e.g. by radioactive methods, e.g. by measuring the incorporation of tritiated thymidine into newly synthesized DNA of the proliferating cells, or by colorimetric methods. Besides cell lines also primary cells have been used to analyze IL-2 activity of immunocytokines. In these assays preactivated peripheral blood lymphocytes or peripheral blood mononuclear cells were incubated with the immunocytokine and activity was determined by means of proliferation or indirectly by the measurement of secondary induced cytokines (Hombach A et al. 2005. Int J Cancer. 115(2), 241-247; Heuser C et al., 2004. Int J Cancer 110(3), 386-394; Hu P et al. 2003. Blood. 101 (12), 4853-4861).

Although these assays are capable of determining the bioactivity of the IL-2 portion, they do not consider that the function of the immunocytokine also depends on the targeting antibody portion. Such assays do not take into account that degradation processes could have occurred, releasing an active cytokine portion. The existing assays would suggest that the molecule is still intact, whereas it was in fact partially degraded and lacking the targeting portion. Therefore, for determination of the bioactivity of the hole, intact protein, an assay is required which assesses the functionality of both moieties. Moreover, the assays have to be highly reliable to fulfil the requirements for drugs applied to humans or animals.

Hank et al. reported that the immunocytokine ch14.18-IL2, a fusion protein of the GD2 specific chimeric antibody ch14.18 and IL-2, was able to stimulate the proliferation of an IL-2 dependent cell line, when bound to GD2 expressing cells. In this assay format, the GD2 expressing tumor cell lines were incubated with immunocytokine on ice, while irradiation of the cells took place (Hank JA et al. 1996. Clin Cancer Res. 2(12), 1951-1959). After washing, cells were cultured with the IL-2 responsive cell line and proliferation was measured by [³H] thymidine incorporation. The authors showed that ch14.18-IL2, when bound to a GD2 expressing tumor cell line, effectively presented IL-2 to the responsive cell line and induced proliferation.

Zhang et al. tested the functional activity of an anti-ovarian carcinoma immunocytokine, composed of an anti-ovarian carcinoma single-chain Fv fused to IL-2 (Zhang et al. 2006. Gynecol Oncol 103(3), 848-852). Irradiated tumor cells expressing the target antigen were incubated with dilutions of the non-purified immunocytokine, washed and incubated with an IL-2 dependent cell line, whose proliferation was determined by [³H] thymidine incorporation. The non-purified immunocytokine was able to stimulate the proliferation of the IL-2 dependent cell line, when bound to antigen-positive cells, whereas no stimulation was observed with antigen-negative cells.

Both studies suggest that stimulation of the IL-2 dependent cells may be achieved by the immunocytokines, bound to antigen-expressing cells. On the other hand, IL-2 receptors have been shown to internalize after IL-2 binding and the question of whether internalization of the IL-2 receptor complex is required for activation has been controversially discussed in the literature. Kumar et al. suggested that the occupancy of the high affinity IL-2 receptor is not sufficient to transduce the T cell growth signal. In their experiments they used an IL-2 antibody of the IgM isotype that blocks IL-2 dependent T cell growth, but not the binding of IL-2, and showed that this antibody inhibits also internalization (Kumar A et al. 1987. J Immunol 139(11), 3680-3684). Results from another similar study using a primary IL-2 antibody and a secondary antibody, capable of crosslinking the primary antibody also indicated that ligand-dependent internalization is coupled to IL-2 dependent proliferation (Duprez V et al. 1991. J Biol Chem 266(3), 1497-1501). In another report the question whether internalization of the IL-2 receptor complex and proliferation are linked processes was addressed more directly (Horwitz JI et al. 1993. Mol Immunol 30(11), 1041-1048). IL-2 was covalently immobilized on an insoluble matrix, thereby preventing free lateral diffusion and internalization of bound IL-2 receptor. The authors observed only limited proliferation of the IL-2 dependent cell line on this matrix. However, the viability of the cells was preserved, indicating that immobilized IL-2 can mediate some of the activity of soluble IL-2 and that internalization of the IL-2 receptor may not be required for at least part of the IL-2 mediated effect. Therefore, it is unclear whether internalization of the IL-2 is a requirement for induction of proliferation although there is some evidence that IL-2 when part of certain fusion proteins does exhibit biological activity.

Analytical evaluation of an immunocytokine dedicated for clinical application requires special demands with regard to assay performance. The methods used in Zhang et al. and Hank et al. cannot fulfil these demands. Both groups use irradiated antigen-expressing cells that are incubated with the respective immunocytokine and with the IL-2 dependent cells. However, antigen expression may vary depending on the cell status (e.g. cell density, growth conditions), leading to an increased variability of the results which cannot be tolerated for drugs used in clinical practice.

Furthermore, additional control experiments are necessary to make sure, that irradiation of antigen expressing cells is complete and that these cells are proliferation-incompetent. Moreover, it cannot be excluded that other surface proteins on these irradiated cells interact with antigens on the IL-2 dependent cell line, thereby potentially influencing proliferation. Conclusively, for analytical evaluation of immunocytokines dedicated for clinical purposes, it is necessary to reduce assay variability to a minimum.

There is therefore a need for reliable bioassays exhibiting low variability which are useful for detecting bioactivity of molecules comprising a targeting part and a part exhibiting biological activity for cells, in particular for immunocytokines.

This is accomplished by a method for the determination of the activity of a molecule,
wherein the molecule comprises at least two active parts, and wherein
the first part is capable of modulating the activity of cells, and
the second part can specifically bind to a target,
comprising following steps:
a) incubating the molecule with the target which is covalently or non-covalently bound to a surface and wherein the target is not bound to the surface via cells expressing the target on the cell surface, wherein the incubation can be performed by incubating the target and the molecule simultaneously, or by consecutive incubation, for example by first incubating the target, and second incubating the molecule.
b) washing
c) adding of cells, wherein the activity of the cells is modulated by the molecule
d) determination of the biological effect on these cells

In a preferred embodiment the biological effect obtained in step d) is compared to the biological effect obtained with a molecule of known activity and/or with an unfused first part capable of modulating the activity of cells. In case the biological effect is compared with a molecule of known activity, the molecule of known activity is preferably analyzed using the same assay. In case the biological effect is compared with an unfused first part, a suitable assay for determining the biological effect of the unfused part is used. Preferably, the same cell line is used.

For fusion proteins comprising a biological active moiety, e.g. the cytokine portion in the immunocytokine, it is often important to compare the biological activity of this moiety to the activity of the non-fused active component; i.e. the unfused first part capable of modulating the activity of cells according to the present invention, e.g. the cytokine alone. This is for example the case for immunocytokines comprising IL-2, as for IL-2 it is still unclear whether internalization is necessary for exerting its effect on IL-2 sensitive cells and/or whether the targeting part interferes with internalization. Moreover, fusing the cytokine to other proteins could lead to conformational changes and thereby potentially affecting biological activity. Finally, the expression system used for protein production as well as protein re-folding, purification and formulation conditions/buffers could influence critical parameters, thereby leading to changes in protein stability and/or function.

In one embodiment, the method is performed once or a several times with a molecule sample, e.g. for quality testing. The method may also be performed more than once, e.g. in order to determine the stability, in particular long-term and short-term stability of a molecule probe.

Moreover, the present assay systems can be used for screening for binding components that inhibit, block or neutralize the function of the molecule, like antibodies and antibody-fragments which inhibit or block or neutralize the function of the portion capable of modulating the activity of cells, in particular the cytokine moiety, in cases the cytokine is part of an immunocytokine. In particular, the present assay can be used to identify the presence of neutralizing antibodies for the molecule in patients.

In another embodiment, the method of the present invention may be used to screen for function-blocking antibodies, which may used as drugs.

"Neutralizing antibodies" are understood as antibodies which reduce or eliminate the biological activity of the molecule of the present invention. This can be determined as described in Gupta et al. 2007. J Immunol Methods 321(1-2), 1-18.

In another embodiment the invention therefore relates to a method for screening for binding components that inhibit, block or neutralize the function of a molecule,
wherein the molecule comprises at least two active parts, and wherein
the first part is capable of modulating the activity of cells, and the second part can specifically bind to a target,
comprising following steps:
x) performing a control experiment as described above
xx) performing an experiment as described above, wherein at least one potential function-inhibiting or neutralizing or blocking component is added at any step prior to step c) of the method described above
xxx) comparing the biological effects obtained in x) and xx)

The first part which is capable of modulating the activity of cells can be for example a cytotoxic agent, which may be linked to a second part covalently either directly or via a peptide or non-peptide-linker. It is well known, that the covalent linkage in immunotoxin conjugates e.g. for conjugates with auristatin-derivatives is sensitive to degradation. Therefore, the methods of the present invention can be used to test whether the intact molecules still exert their effect on the cells.

The biological effect on cells can be tested by measurement of proliferation, cell viability, cell death, apoptosis or inhibition of protein biosynthesis, depending on the biological active portion.

In one embodiment of the present invention, the molecule used in the methods is an immunotoxin or immunoconjugate.

In a preferred embodiment of the present invention, the molecule is a fusion protein. Fusion proteins are especially sensitive, as all of the two or more domains of the molecule need to be folded in a distinct way and fusion could therefore interfere with the activity of all parts.

In a preferred embodiment of the present invention, the first part of the molecule is a cytokine, in particular selected from IL-1, IL-2, IL-12, IL-15, GM-CSF, G-CSF, VEGF, IFNγ and TNFα.

In fusion proteins of the present invention, the first part may be located either N-terminally or C-terminally of the second part. For example, in L19-IL2, the IL-2 part, which refers to the first part to the molecule, is located C-terminally of the L19-antibody fragment part, which refers to the second part of the molecule according to the present invention.

In especially preferred embodiments, the cytokine is IL-2, in particular human IL-2. The term "human IL-2" encompasses wildtype human IL-2 as well as functional variants thereof which exhibit the same activity as wildtype human IL-2. In particular, one or more amino acids can be modified without affecting biological activity; e.g. the N-terminal methionine may be missing. Also, the human IL-2 may be glycosylated, partially glycosylated or unglycosylated.

A particularly preferred human IL-2 as first part of the molecule of the invention is a IL-2 having the sequence of SEQ ID No. 1.

A particularly preferred human TNFα as first part of the molecule of the invention is a TNFα having the sequence of SEQ ID No. 2.

The second part of the molecules of the present invention can specifically bind to a target.
In a preferred embodiment of the present invention the second part of the molecule is selected from an antibody, antibody fragment or antibody mimetic.

"Specifically binding" as used herein refers to binding to the corresponding target. Typically, the second part of the molecule, antibody, antibody fragment or antibody mimetic binds with an affinity of at least about 1x10⁻⁷ M, preferably of at least about 1x10⁻⁹ M, and binds to the predetermined target with an affinity that is at least two-fold greater than its affinity for binding to a non-specific target (e.g. BSA, casein) other than the predetermined target or a closely-related target.

"Antibody" as used herein encompasses full length antibodies, comprising native antibodies, monoclonal antibodies, polyclonal antibodies and multispecific antibodies (e.g., bispecific antibodies), human antibodies, humanized antibodies, chimeric antibodies, and full IgG antibodies.

The term "antibody fragment" refers to a portion of a full length antibody, in which a variable region or a functional capability is retained, namely the specific binding to the target. Examples of antibody fragments include, but are not limited to, a Fab, Fab', F(ab')2, Fd, Fv, scFv and scFv-Fc fragment, a diabody, a linear antibody, small immunoprotein formats, a single-chain antibody, a minibody, a diabody formed from antibody fragments, and multispecific antibodies formed from antibody fragments. Antibody fragments are usually smaller than full antibodies. Thereby, the pharmacokinetics are different and some antibody fragments only consist of one polypeptide chain, which can make production easier. However, such fusion proteins comprising antibody fragments often suffer from a reduced stability. Preferably, the antibody fragment is in scFv, (scFv)2, or small immunoprotein format. The small immunoprotein format can be a format based on a CH3-domain (for example described in US 5,837,821) or εs2CH4-domain of human IgE (for example described in WO 03/076469).

The term "monoclonal antibody" (mAb) refers to an antibody obtained from a population of substantially homogeneous antibodies; that is, the individual antibodies comprising the population that are identical except for naturally occurring mutations that may be present in minor amounts. Monoclonal antibodies are highly specific, being directed against a single antigenic determinant, also referred to as an epitope. The modifier "monoclonal" is indicative of a substantially homogeneous population of antibodies directed to the identical epitope and is not to be construed as requiring production of the antibody by any particular method. Monoclonal antibodies can be made by any technique or methodology known in the art; for example, the hybridoma method first described by Koehler et al., 1975, Nature 256:495, or recombinant DNA methods known in the art (see, e.g., U.S. Patent No. 4,816,567). In another example, monoclonal antibodies can also be isolated from phage antibody libraries, using techniques described in Clackson et al., 1991, Nature 352: 624-628, and Marks et al., 1991, J. Mol. Biol. 222: 581-597.

In contrast, the antibodies in a preparation of polyclonal antibodies are typically a heterogeneous population of immunoglobulin isotypes and/or classes and also exhibit a variety of epitope specificity.

The term "chimeric" antibody as used herein is a type of monoclonal antibody in which a portion of or the complete amino acid sequence in one or more regions or domains of the heavy and/or light chain is identical with, homologous to, or a variant of the corresponding sequence in a monoclonal antibody from another species or belonging to another immunoglobulin class or isotype, or from a consensus sequence.

Certain types of antibody fragments can be generated by enzymatic treatment of a full-length antibody. Papain digestion of antibodies produces two identical antigen-binding fragments called "Fab" fragments, each with a single antigen-binding site, and a residual "Fc" fragment, so called because of its ability to crystallize readily. The Fab fragment also contains the constant domain of the light chain and the CH1 domain of the heavy chain. Pepsin treatment yields a F(ab')2 fragment that has two antigen-binding sites and is still capable of cross-linking antigen.

Fab' fragments differ from Fab fragments by the presence of a few additional residues at the C-terminus of the CH1 domain, including one or more cysteines from the antibody hinge region. Fab-SH is the designation herein for a Fab' in which the cysteine residue(s) of the constant domains bear a free thiol group. F(ab')2 antibody fragments are pairs of Fab' fragments linked by cysteine residues in the hinge region. Other chemical couplings of antibody fragments are also known.

"Fv" is a minimum antibody fragment that contains a complete antigen-recognition and binding site consisting of a dimer of one heavy and one light chain variable domain in tight, non-covalent association. In this configuration, the three CDRs of each variable domain interact to define an antigen-binding site on the surface of the VH VL dimer. Collectively, the six CDRs confer antigen-binding specificity to the antibody.

A "single-chain Fv" or "scFv" antibody fragment is a single chain Fv variant comprising the VH and VL domains of an antibody, in which the domains are present in a single polypeptide chain and which is capable of recognizing and binding antigen. The scFv polypeptide optionally contains a polypeptide linker positioned between the VH and VL domains that enables the scFv to form a desired three-dimensional structure for antigen binding (see, e.g., Pluckthun, 1994, In The Pharmacology of Monoclonal Antibodies, Vol. 113, Rosenburg and Moore eds., Springer-Verlag, New York, pp. 269-315).

The term "diabodies" refers to small antibody fragments having two antigen-binding sites. Each fragment contains a heavy chain variable domain (VH) concatenated to a light chain variable domain (VL). By using a linker that is too short to allow pairing between the two domains on the same chain, the linked VH-VL domains are forced to pair with complementary domains of another chain, creating two antigen-binding sites.

Diabodies are described more fully, for example, in EP 404,097; WO 93/11161; and Hollinger et al., 1993, Proc. Nat. Acad. Sc. USA 90: 6444-6448.

A humanized antibody or a humanized antibody fragment includes an immunoglobulin amino acid sequence variant, or fragment thereof, which is capable of binding to a predetermined antigen and which, comprises one or more framework regions (FRs) having substantially the amino acid sequence of a human immunoglobulin and one or more CDRs having substantially the amino acid sequence of a non-human immunoglobulin. This non-human amino acid sequence is referred to herein as an "import" sequence, which is typically taken from an "import" antibody domain, particularly a variable domain. In general, a humanized antibody includes at least the CDRs or HVLs of a non-human antibody, inserted between the FRs of a human heavy or light chain variable domain.

"Native antibodies" are defined herein as heterotetrameric glycoproteins, typically of about 150,000 daltons, composed of two identical light (L) chains and two identical heavy (H) chains. Each light chain is covalently linked to a heavy chain by one disulfide bond to form a heterodimer. The heterotetramer is formed by covalent disulfide linkage between the two identical heavy chains of such heterodimers. Although the light and heavy chains are linked together by one disulfide bond, the number of disulfide linkages between the two heavy chains varies by immunoglobulin isotype. Each heavy and light chain also has regularly spaced intrachain disulfide bridges. Each heavy chain has at the amino-terminus a variable domain (VH), followed by three or four constant domains (CH1, CH2, CH3, and CH4), as well as a hinge region between CH1 and CH2. Each light chain has two domains, an amino-terminal variable domain (VL) and a carboxy-terminal constant domain (CL). The VL domain associates non-covalently with the VH domain, whereas the CL domain is commonly covalently linked to the CH1 domain via a disulfide bond. Particular amino acid residues are believed to form an interface between the light and heavy chain variable domains (Chothia et al., 1985, J Mol. Biol. 186:651-663.) The term "hypervariable" refers to the fact that certain sequences within the variable domains differ extensively in sequence among antibodies and contain residues that are directly involved in the binding and specificity of each particular antibody for its specific antigenic determinant. Hypervariability, both in the light chain and the heavy chain variable domains, is concentrated in three segments known as complementarity determining regions (CDRs) or hypervariable loops (HVLs). CDRs are defined by sequence comparison in Kabat et al., 1991, In: Sequences of Proteins of Immunological Interest, 5th Ed. Public Health Service, National Institutes of Health, Bethesda, MD., whereas HVLs are structurally defined according to the three-dimensional structure of the variable domain, as described by Chothia and Lesk, 1987, J. Mol. Biol. 196: 901-917.

Where these two methods result in slightly different identifications of a CDR, the structural definition is preferred. As defined by Kabat, CDR-L1 is positioned at about residues 24-34, CDR-L2, at about residues 50-56, and CDR-L3, at about residues 89-97 in the light chain variable domain; CDR-H1 is positioned at about residues 31-35, CDR-H2 at about residues 50-65, and CDR-H3 at about residues 95-102 in the heavy chain variable domain.

The term "label" refers to a detectable compound or composition that is conjugated directly or indirectly to the antibody. The label may itself be detectable (e.g., radioisotope labels or fluorescent labels) or, in the case of an enzymatic label, may catalyze chemical alteration of a substrate compound or composition that is detectable.

Although fibronectins (FNs) are the product of the single FN gene, the resulting protein can exist in multiple forms which - apart from posttranslational modifications - arise from alternative splicing of its primary RNA transcript. This polymorphism which leads to as many as 20 different isoforms in human FN, thereby generating FNs with different solubility, cell adhesive and ligand-binding properties, provides cells with the possibility to modify the composition of the extracellular matrix (ECM) in a tissue-specific manner. Alternative splicing takes place in three regions of the primary RNA transcript: Exon usage or skipping leads to either inclusion or omission of two type-III repeats, extra-domain B (EDB, also termed EIIIB or EDII), which is inserted between FN type-III repeats III7 and III8, or/and extra-domain A (EDA, also termed EIIIA or EDI), inserted between FN type-III repeats III11 and III12. This type of splicing occurs in many vertebrates, including Xenopus, chicken, rat, dog and human.

"ED-B" domain" is to be understood as the extra-domain B of human fibronectin. It is often referred to as EDB, EIIIB or EDII.

"7-B-8-9" or "7B89" is to be understood as the peptide corresponding to the domains FN type-III repeat III7, ED-B and FN type-III repeats III8 and III9 (N to C terminus).

"Antibody mimetics" are understood as binding molecules based on protein frameworks ("scaffolds") which specifically bind to the target and which are distinct from antibodies and antibody fragments. Such scaffolds are described in Binz et al., 2005, Nat. Biotechnol. 23, 1257-1268. Antibody mimetics specifically binding to ED-B FN are described in Grabulovski et al., J. Biol. Chem., 2007, 282:3196-3204.

In an especially preferred embodiment of the invention, the target which is specifically bound by the second part of the molecule, is ED-B fibronectin or parts thereof comprising the ED-B domain.
Particularly preferred is the use of the peptide comprising the domains 7-B-8-9 of ED-fibronectin, in particular the peptide having the sequence of the domains 7-B-8-9 of ED-fibronectin.

In an especially preferred embodiment of the invention, the antibody or antibody fragment comprises the CDR regions of L19 and/or comprise at least one Vh and at least one VI chain of the L19-antibody. In particular, the L19-antibody fragment is in scFv format. In a more preferred embodiment the L19-antibody fragment has the CDR sequences of L19. The CDR sequences of L19 have sequences according to SEQ ID No. 5 to 10. Even more preferred, it has a Vh and a VI chain of L19. In particular, the Vh and VI chains of L19 have sequences according to SEQ ID No. 3 and 4. In a preferred embodiment Vh and VI of L19 are linked by a linker peptide according to SEQ ID No. 12.

In case the molecule is a fusion protein comprising IL-2, the cells are IL2-responsive cells.

In a preferred embodiment, the cells responsive to IL-2 are selected from CTLL-2, HT-2 and/or TF1 β-cells. In another embodiment the IL-2 responsive cell lines are selected from A9.12 (Winandy M et al. 1998. J Immunol Methods 215(1-2), 81-94), ATH8 (Antonen J et al. 1987. J Immunol Methods 99(2), 271-275), FD.C/2 (Le Gros GS et al. 1985. J Immunol 135(6), 4009-4014), Kit 225 (Hori T et al. 1987. Blood 70(4), 1069-1072) and Sez 627 (Namiuchi S et al. 1986. J Immunol Methods 94(1-2), 215-224). In yet another embodiment, primary cells can be used.

In an especially preferred embodiment, the IL-2 responsive cells are CTLL-2 cells.

In a preferred embodiment of the present invention, the biological activity of the cells which is determined, is selected from stimulation or inhibition of cell proliferation, cytotoxicity, viability, cell death, protein biosynthesis and differentiation. Stimulation or inhibition of cell proliferation, cytotoxicity, viability, cell death, protein biosynthesis and differentiation can be determined colorimetrically, fluorometrically, radioactively and/or by microscopy. For example, proliferation can be determined radioactively, using [³H]-labeled thymidine. A method for determining proliferation colorimetrically is shown in the examples. Also, proliferation or differentiation may be determined by microscopy, whereby the density of cells and/or the cell differentiation may be determined. Cell differentiation may also be measured by determining cell differentiation markers, typically using antibodies directed against them. The bound antibodies in turn may then be detected by a label attached to the primary antibody or by a secondary antibody with an appropriate detectable label. Such label may again be a radioactive, fluorescent, or enzymatically active label.

In a preferred embodiment, the detection limit for the activity of human IL-2 is low enough in order to ensure that the assay can be used to analyze molecules to be used in patients.

In another preferred embodiment, the standard deviation/error for the determination of the IL-2 activity is low enough in order to make the assay suitable to analyze molecules to be used in patients.

In a preferred embodiment, the methods of the present invention fulfil the requirements for quality testing of drugs.

In a particularly preferred embodiment, the present invention relates to a method for the determination of the activity of the L19-IL2 fusion protein, comprising following steps:
- incubating L19-IL2 with the ED-B fibronectin or parts thereof comprising the ED-B domain which is covalently or non-covalently bound to a surface
- washing
- adding of IL-2-responsive cells, preferably selected from CTLL-2, HT-2 and/or TF1β cells
- determination of cell viability.

In a preferred embodiment, the biological effect obtained by the method is compared to the biological effect of unfused IL2 in an assay suitable for determining the biological effect of IL2 and/or to L19-IL2 of known activity. L19-IL2 of known activity is preferably analyzed under the same conditions as L19-IL2 to be tested. Preferably, both probes are tested in parallel, or in sequence.

The determination of cell viability is preferably determined after an incubation period at about 37°C. Typically, the incubation time is between about 12 hours and about 50 hours.

In particular, the L19-IL2 protein comprises the SEQ ID No. 5 to 10 and/or 3 and 4. In particular, the L19-IL2 protein comprises the SEQ ID No. 1.

In a preferred embodiment, L19 and IL-2 are linked by a linker peptide, preferably according to SEQ ID No. 11.

In a preferred embodiment, the part of ED-B fibronectin comprising the ED-B domain is 7B89.

In another preferred embodiment the target is ED-B fibronectin or comprises 7B89, in particular is 7B89.

In a preferred embodiment, cell viability is determined colorimetrically.

The present invention moreover relates to kits which can be used for the methods of the present invention.

In one embodiment, the invention relates to a Kit, comprising
1) a surface covalently or non-covalently coated with a target
2) a molecule,
wherein the molecule comprises at least two active parts, and wherein
the first part is capable of modulating the activity of cells, and the second part can specifically bind to a target.

In a preferred embodiment, the target is ED-B fibronectin or parts thereof comprising the ED-B domain.

Non-covalent attachment of the target to the surface may be achieved by biotin covalently attached to the target and the surface being coated with streptavidin. Similar coating systems are well known to the person skilled in the art (e.g glutathione-coated surfaces for GST-tagged proteins/peptides or nickel-chelate coated surfaces for histidine-tagged proteins/ peptides).

Covalent attachment of the target to the surface may be achieved via plates with a secondary amino group-coupled surface.

Preferably, the surface is a microtitre plate.

Preferably, the kit in addition contains cells, wherein the activity of the cells can be modulated by the molecule.

In another preferred embodiment, the kit in addition contains reagents for the determination of the viability or proliferation of the cells.

### Figures and Tables:

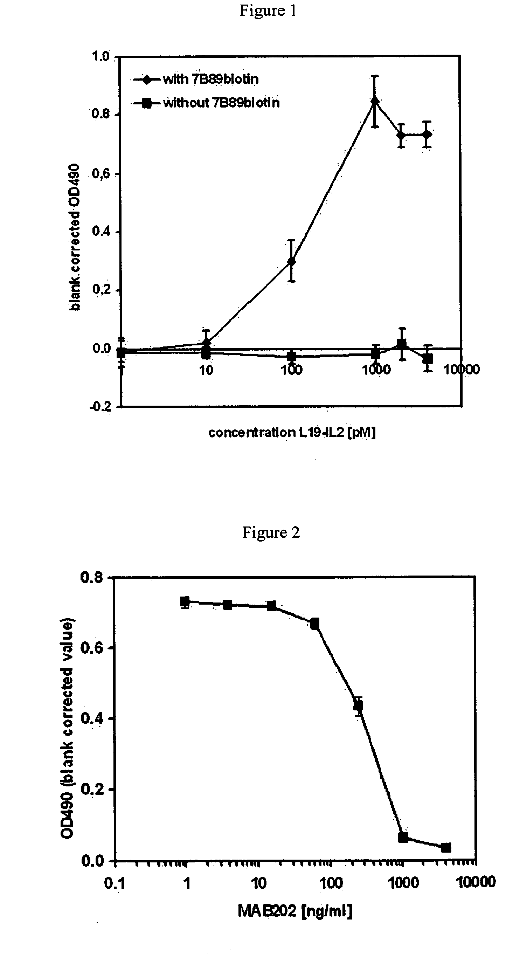

Figure 1 shows the proof of principle for the sandwich IL-2 bioactivity assay. Blank corrected absorbance values at 490nm are plotted against the L19-IL2 concentrations. L19-IL2 dilutions were incubated with and without the biotinylated 7B89 fragment (7B89biotin) on a streptavidin-coated microtitration plate. After washing approximately 43000 CTLL-2 cells/well were added and plates were incubated at 37°C (5% CO₂) for about 24h. Development, data measurement and analysis was performed as described in the methods section. Data points represent blank corrected mean values of triplicates (± standard deviation)
Figure 2 shows specificity of the assay format. Blank corrected absorbance values at 490nm are plotted against the L19-IL2 concentrations. L19-IL2 (2.1ng/well), 7B89biotin (25ng/well) and different dilutions of the IL-2 specific neutralizing antibody MAB202 were incubated on a streptavidin-coated plate, the plate was washed and CTLL-2 cells (approximately 40000cells/well) were added. After 20h at 37°C (5% CO₂) the plate was developed and OD490 was measured. Blank corrected mean values of triplicates (± standard deviation) of one representative experiment are reported.

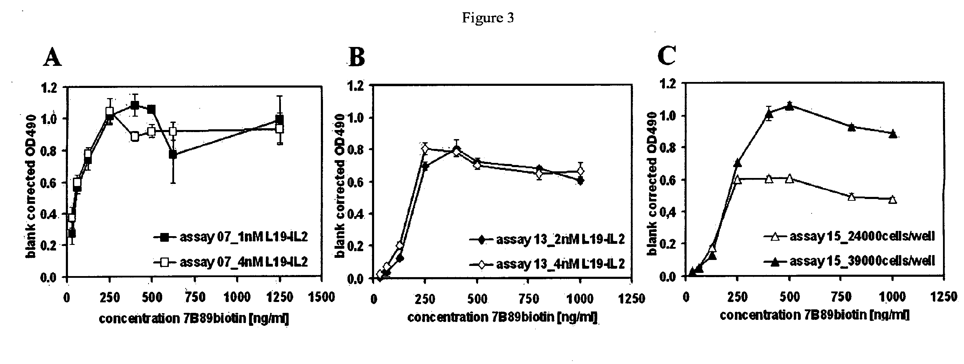

Figure 3 demonstrates the finding of a suitable 7B89biotin concentration. Blank corrected absorbance values at 490nm are plotted against the 7B89biotin concentrations. A, B, C represent three tests using different experimental conditions: A: 40000cells/well, 22.5h incubation at 37°C, 1nM/4nM L19-IL2; B: 26500cells/well, 44h incubation at 37°C, 2nM/4nM L19-IL2; C: 24000/39000cells/well, 43.25h incubation at 37°C, 1nM L19-IL2. Maximum signal was obtained using 7B89biotin concentrations of 250 to 500ng/ml, whereas the signal dramatically decreased at lower 7B89biotin concentrations. A concentration of 400 to 500ng/ml 7B89biotin was regarded suitable. Data points represent blank corrected mean values of triplicates (± standard deviation).

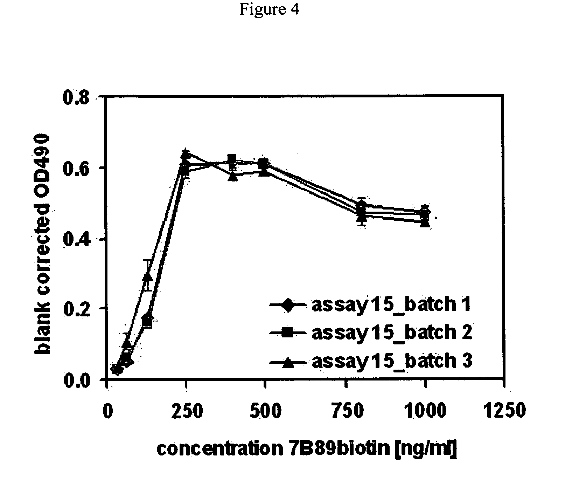

Figure 4 shows the comparison of different 7B89biotin batches. Blank corrected mean absorbance values at 490nm are plotted against the 7B89biotin concentrations. L19-IL2 (1 nM) was incubated with different concentrations 7B89biotin on streptavidin-coated microtitration plates. After washing approximately 24000 CTLL-2 cells/well were added and plates were incubated at 37°C for about 43.25h. Development, data measurement and analysis was performed as described in the methods section. Data points represent blank corrected mean values (± standard deviation) of duplicates.

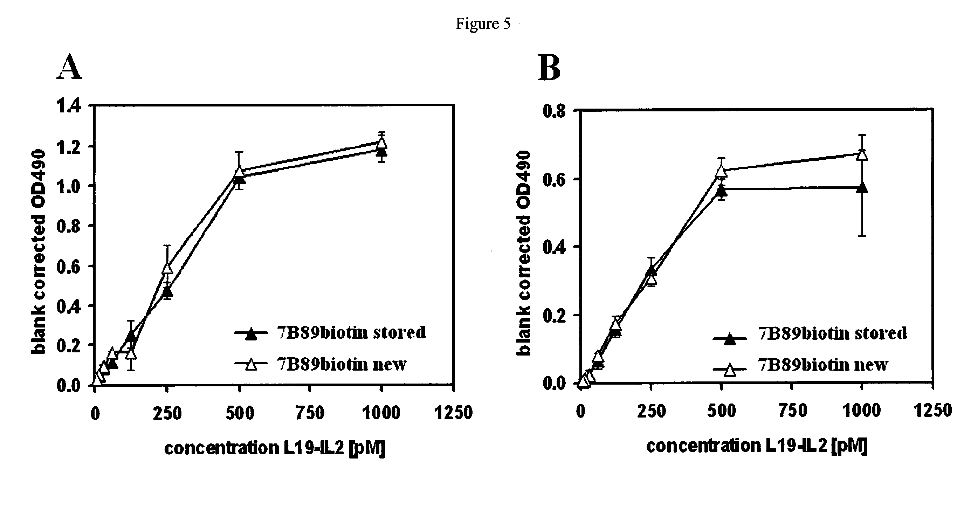

Figure 5 shows evaluation of 7B89biotin stability on 4°C. Blank corrected absorbance values at 490nm are plotted against L19-IL2 concentrations. L19-IL2 and 7B89biotin were incubated on streptavidin-coated microtitration plates. Experiments were performed with a 7B89biotin aliquot that had been freshly thawed and stored at 4°C for 15-16 weeks, respectively (A: 7B89biotin new (freshly thawed); 7B89biotin stored (stored at 4°C for 15 weeks); B: 7B89biotin new (stored at 4°C for 8 days); 7B89biotin stored (stored at 4°C for 16 weeks)) After washing approximately 40000 CTLL-2 cells/well were added and plates were incubated on 37°C for 19.5-20.5h. Development, data measurement and analysis was performed as described in the methods section. Data points represent mean values (± standard deviation) of triplicates.

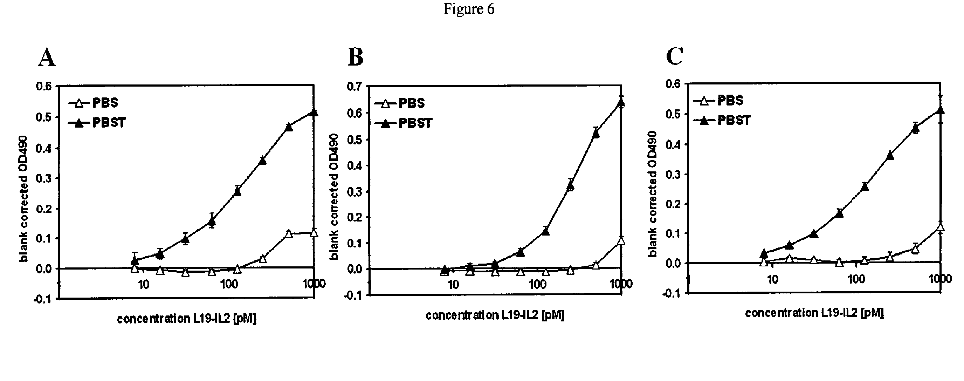

Figure 6 shows comparison of different washing buffers. Blank corrected absorbance values at 490nm are plotted against L19-IL2 concentrations. After incubation of L19-IL2 and 7B89biotin, plates were washed with PBS and PBS/0.05% v/v Tween 20 (PBST), respectively, and subsequently washed twice with cell culture medium. Then, approximately 30000-40000 CTLL-2 cells/well were added and plates were incubated on 37°C for 20.5-24h. Development, data measurement and analysis was performed as described in the methods section. Data points of the three experiments (A, B, C) represent mean values (± standard deviation) of duplicates (C) or triplicates (A, B).

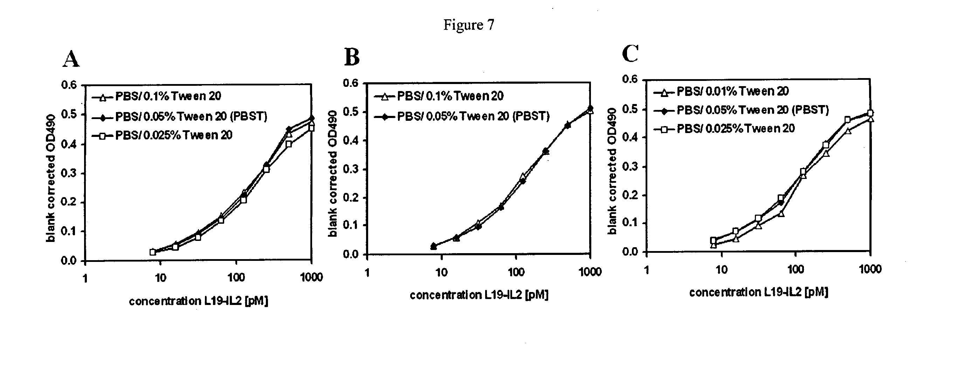

Figure 7 shows comparison of washing buffers containing different Tween 20 concentrations. Blank corrected absorbance values at 490nm are plotted against L19-IL2 concentrations. After incubation of L19-IL2 and 7B89biotin, plates were washed with PBS containing the indicated concentrations of Tween 20 and subsequently washed twice with cell culture medium. CTLL-2 (30000-40000 cells/well) were added and plates were incubated on 37°C for 20.5-21 h. Development, data measurement and analysis was performed as described in the methods section. Data points of the three experiments (A, B, C) represent mean values of duplicates.

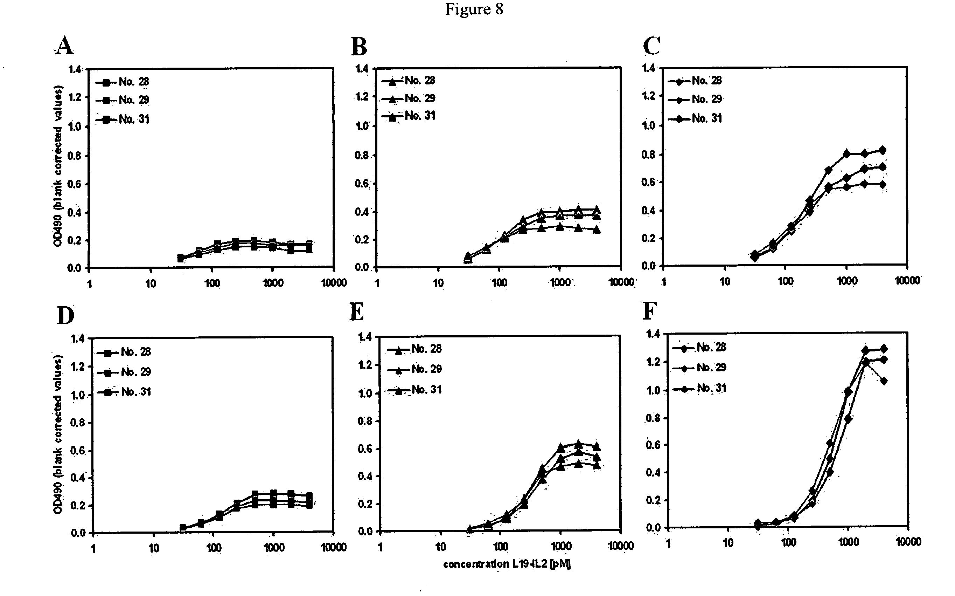

Figure 8 shows optimization of CTLL-2 concentration and incubation time at 37°C. Blank corrected absorbance values at 490nm are plotted against L19-IL2 concentrations. After preincubation with 7B89biotin and L19-IL2 approximately 10000 (A/D), 20000 (B/E) and 40000 (C/F) cells were seeded per well. Plates were incubated at 37°C in a humidified incubator for about 18-22h (A/B/C) or 42-45h (D/E/F), development solution was added and OD490 was determined after 4h. Mean values of duplicates for three experiments (No. 28, 29, 31) are reported.

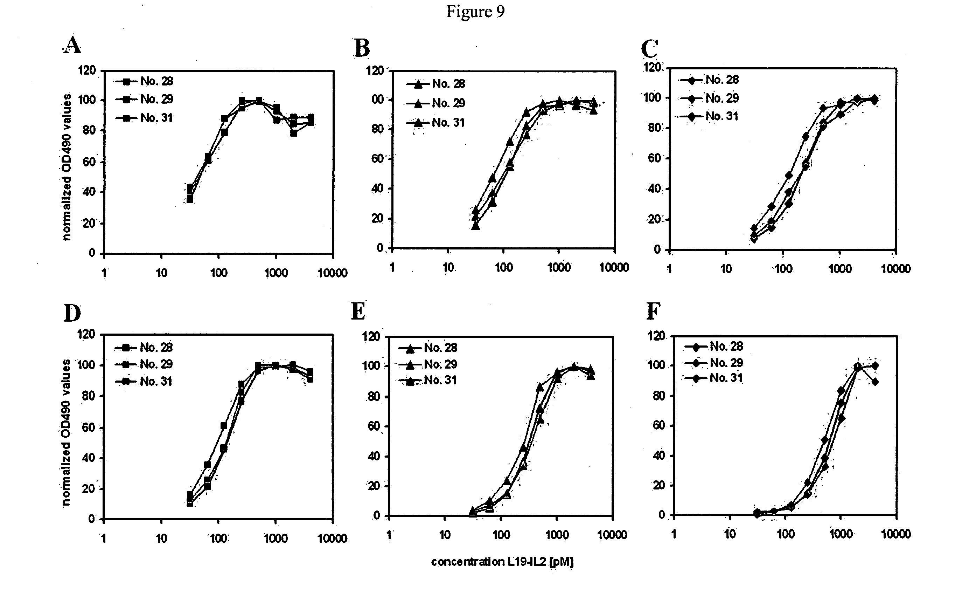

Figure 9 shows optimization of CTLL-2 concentration and incubation time at 37°C. To consider assay-to-assay variability same results depicted in Figure 8 were normalized and normalized values were plotted against L19-IL2 concentrations.

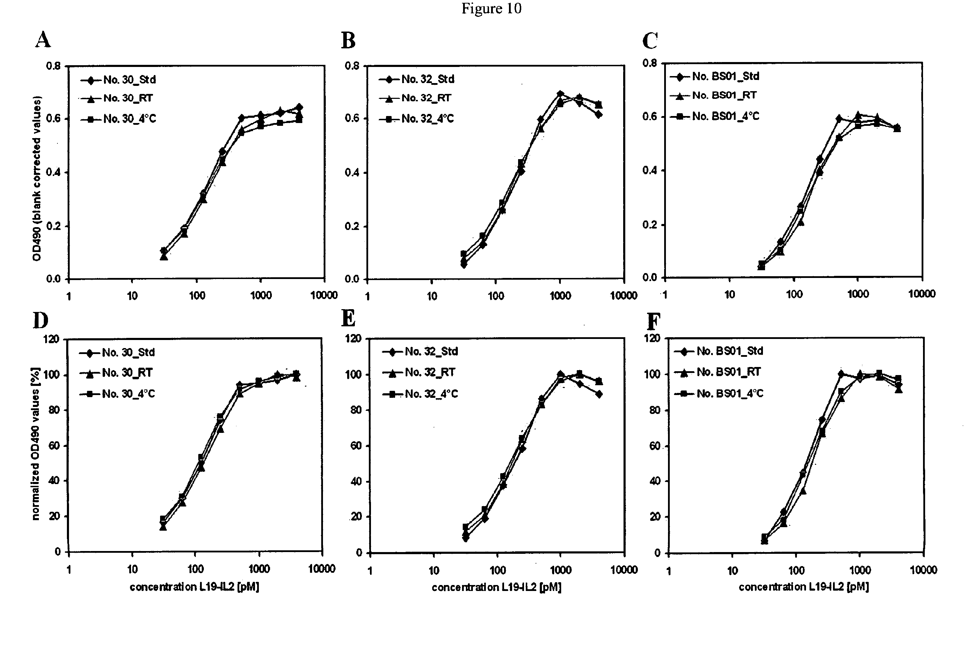

Figure 10 shows short time stability of L19-IL2 at 4°C and room temperature. L19-IL2 (8nM in PBST) was stored overnight (-17-19h) at 4°C and room temperature (RT), respectively. This 8nM sample was serially diluted twofold seven times. Additionally, a dilution series was freshly prepared from the stock solution and used as standard curve (Std). Three assays (No. 30, 32, BS01) were performed with an incubation time at 37°C of approximately 20h. Blank corrected mean OD490 values from duplicates (A/B/C) and normalized values (D/E/F) are reported.

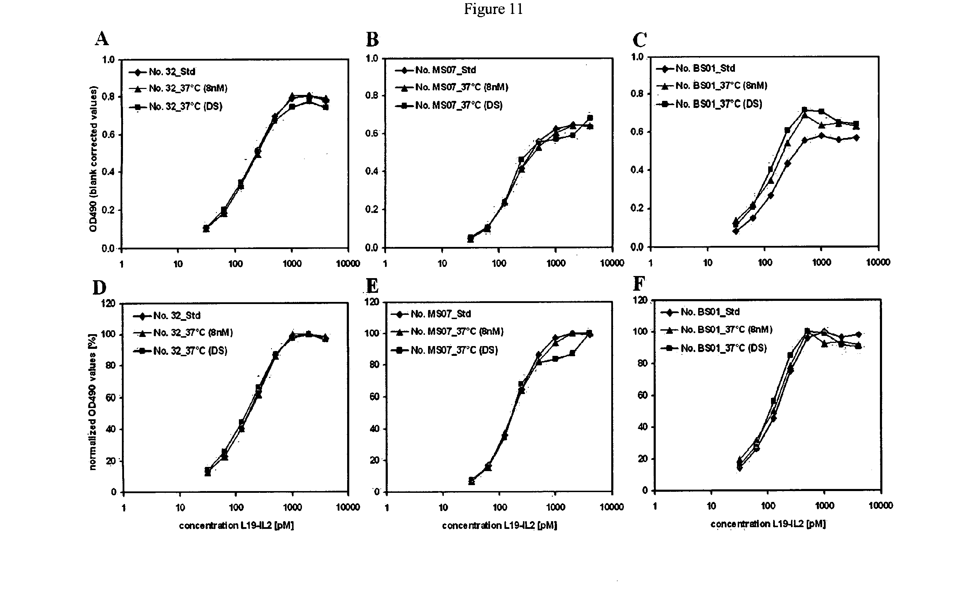

Figure 11 shows short time stability of L19-IL2 at 37°C. L19-IL2 (8nM) and an L19-IL2 dilution series (DS) were stored overnight (∼17-19h) at 37°C in a humidified incubator. This 8nM sample was serially diluted twofold seven times. Additionally, a dilution series was freshly prepared from the stock solution and used as standard curve (Std). Three assays (No. 32, MS07, BS01) were performed with an incubation time at 37°C of approximately 20h. Blank corrected OD490 values from duplicates (A/B/C) and normalized values (D/E/F) are reported.

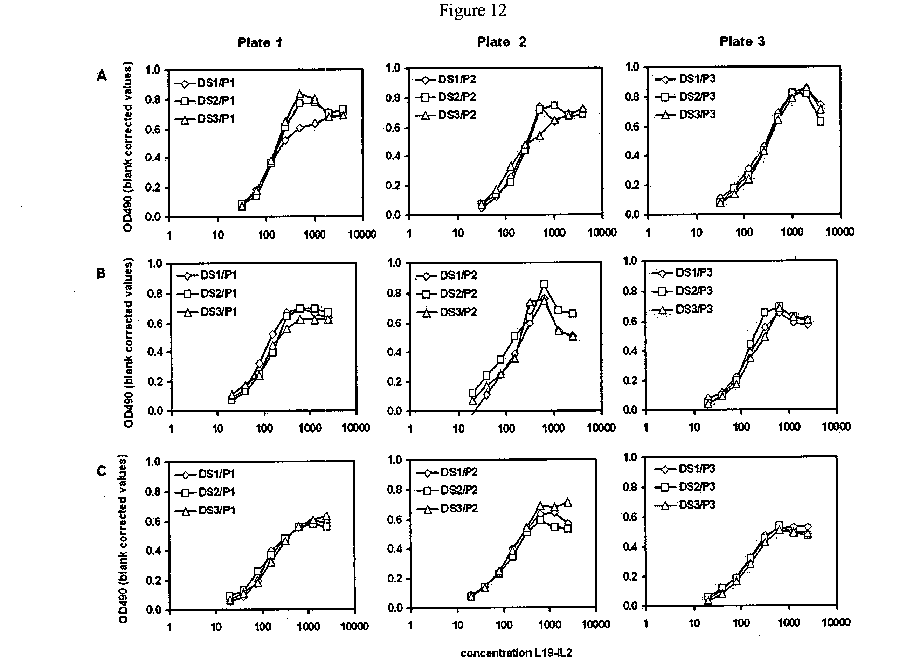

Figure 12 shows preliminary data on intraplate variability for the sandwich IL-2 bioactivity assay. Three experiments (A/B/C) with each three plates were performed. For each experiment three independent L19-IL2 dilution series were made and applied in duplicate on different plate positions. The plates were incubated with about 40000 cell per well at 37°C 5% CO₂ for 20h, then the development solution was added and OD490 was determined after 4h. DS, dilution series; P, plate. Blank corrected mean values of duplicates are depicted.

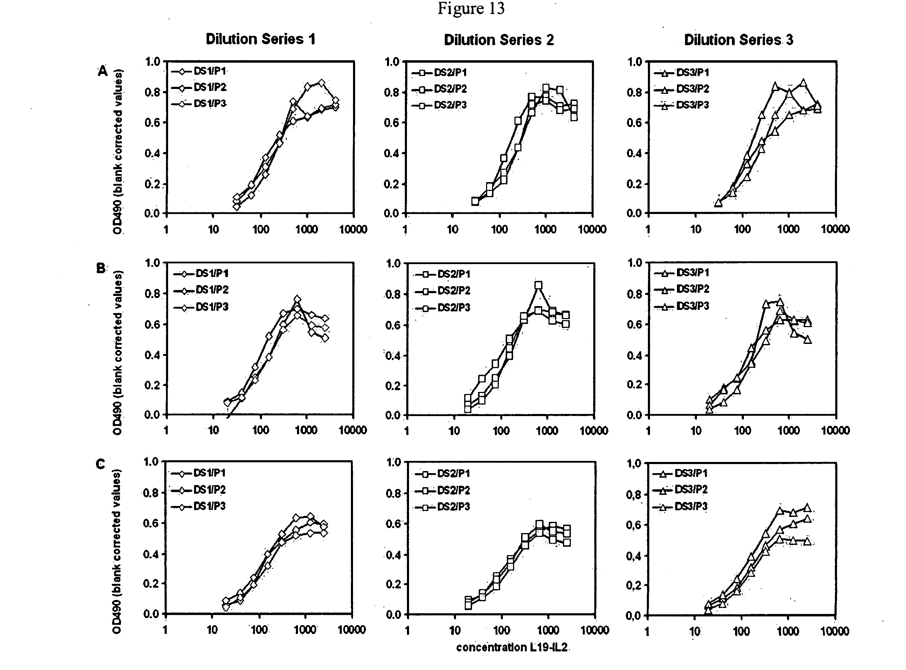

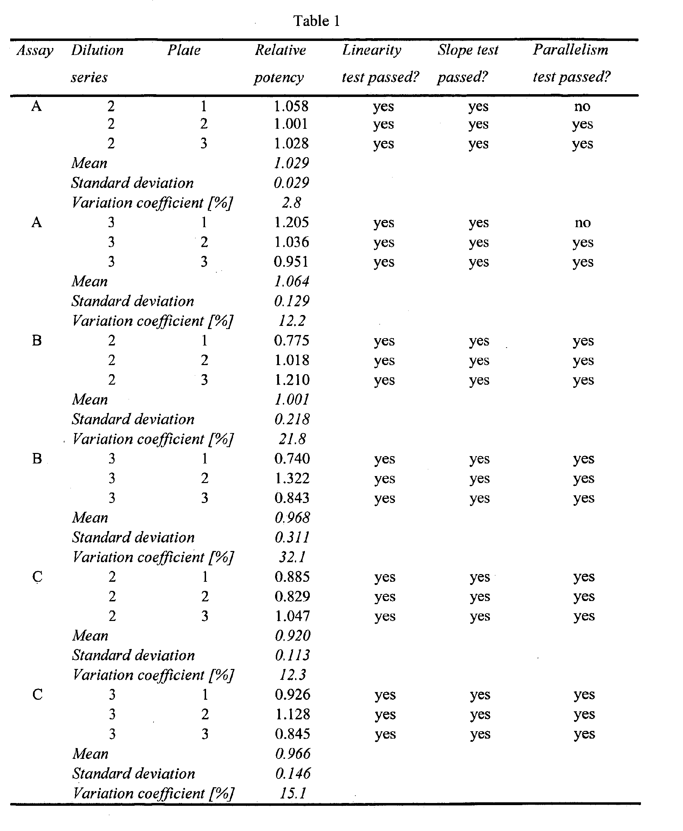

Figure 13 shows preliminary data on interplate variability for the sandwich IL-2 bioactivity assay, based on values that are also depicted in Figure 12.

Table 1 shows preliminary results of parallel-line analysis and calculated potency values for three experiments. Absorbance values of the experiments depicted in Figure 12 and Figure 13 were used to calculate potency of L19-IL2 and to perform parallel-line analysis.

### Examples

### Materials & Methods

### Culture and preparation of CTLL-2 cells

The IL-2 dependent murine cytotoxic T-cell line CTLL-2 (ATCC) was maintained in CTLL-2 growth medium (RPMI 1640 +L-Glutamine -NaHCO₃; Gibco) supplemented with 2.5g/l D-Glucose, 2mM L-Glutamine, 2mM Sodium Pyruvate, 10mM HEPES, 1.5g/l Sodium Bicarbonate, 10% foetal bovine serum and 10% T-Stim Culture Supplement with ConA (BD Biosciences). Three times a week cells were diluted in fresh medium, usually to 7000-15000cells/ml. Cell density was kept below approximately 400000cells/ml, as at high concentrations cells rapidly begin to loose viability due to IL-2 deprivation. For bioassay experiments cells were washed three times with CTLL-2 medium (without T-Stim), counted and cell concentration was adjusted to the respective cell density.

### Sandwich IL-2 bioactivity assay

L19-IL2 (50µl) and 7B89biotin (50µl), diluted in PBST (PBS/ 0.05% Tween 20) were incubated on Streptavidin-coated Immobilizer F96 MicroWell Plates (Nunc) at room temperature for about 2-2.5h under continuous shaking. Control wells were incubated only with PBST (100µl). The contents of the wells were aspirated and plates were washed two times with PBST and subsequently two times with CTLL-2 medium (without T-Stim). After washing, CTLL-2 cells that had been washed three times with CTLL-2 medium (without T-Stim) were added. Control wells were supplemented with 10% T-Stim (with ConA). Plates were incubated at 37°C in a humidified incubator (5% CO₂) for one to two days, then CellTiter96 Aqueous One Solution (Promega) was added and plates were again incubated at 37°C for 4h. Absorbance at 490nm was measured and used for data analysis. Data anaylsis was performed using Microsoft Excel 2003 and PLA 1.2.06 (Stegmann Systemberatung) for parallel-line analysis.

### Results

### Sandwich IL-2 bioactivity assay: Assay design

The assay design includes two major steps, in the first of which the biotinylated antigen 7B89biotin and L19-IL2 itself are incubated on a streptavidin-coated microtitration plate. The antigen comprises the fibronectin domains 7, 8, 9 and ED-B (Carnemolla B et al. 1996. Int J Cancer 68(3), 307-405) and its biotinylated form was designated 7B89biotin. After washing CTLL-2 cells are added in the second step and the plate is incubated at 37°C in a humidified incubator. Development solution is then added, the plate is further incubated at 37°C, 5% CO₂ and read out is performed colorimetrically. Proof of principle and specificity of the sandwich IL-2 bioactivity assay CTLL-2 cells incubated with L19-IL2 and 7B89biotin showed increasing viability with increasing concentrations of L19-IL2, whereas no signal over background level was observed without 7B89biotin. Thus, 7B89biotin was essential for binding of L19-IL2 to the plate and for allowing to exert its biological activity (Figure 1).
Specificity of the sandwich IL-2 bioactivity assay was proven by neutralization experiments with the anti-human IL-2 antibody MAB202 (clone: 5334; R&D systems), which has been reported to neutralize human IL-2 bioactivity (Figure 2). L19-IL2, biotinylated 7B89 and dilutions of the monoclonal IL-2 antibody were incubated on a streptavidin-coated plate. After washing fresh medium and CTLL-2 cells were added, plates were incubated at 37°C in a humidified incubator for 20h and developed by addition of the CellTiter96 Aqueous One Solution. Increasing concentrations of the IL-2 antibody resulted in a decreased viability of the CTLL-2 cells, demonstrating that cell survival was strictly dependent on the presence of the recombinant protein in its active form and not from other factors in the medium or buffer. Thus, the assay was specific for L19-IL2 bioactivity.

### Concentration range for 7B89biotin

For determination of a suitable 7B89biotin concentration range, the bioassay was performed using different 7B89biotin concentrations (Figure 3). Although experimental conditions varied, the maximum signal was obtained using 7B89biotin concentration of 250-500ng/ml, whereas the signal dramatically decreased at lower 7B89biotin concentrations. Additionally, different batches of 7B89biotin were compared in order to analyze general applicability of the selected concentration range (Figure 4). Comparable results were obtained with all three tested batches, confirming that 250-500ng/ml 7B89biotin represents a suitable concentration range for this assay format. Stability of 7B89biotin on 4°C
To evaluate stability of 7B89biotin, experiments were performed using 7B89biotin stock solution that had been stored at 4°C for different time periods (Figure 5). Even after 15-16 weeks at 4°C 7B89biotin could be used in the IL-2 bioactivity assay without major loss of functional activity.

### Determination of washing parameters

After incubation of 7B89biotin and L19-IL2 on a streptavidin-coated microtitration plate, several washing steps with buffer and cell culture media are performed to remove unbound material. To optimize this procedure different washing conditions were compared. First, the effect of Tween 20 in phosphate buffered saline (PBS) was analyzed by comparing PBS and PBS containing 0.05% Tween 20 (PBST) (Figure 6). In three independent experiments it was shown that cell viability was almost completely lost, if PBS lacking Tween 20 was used for washing. Thus, Tween 20 is indispensable for the washing procedure.
Next, different Tween 20 concentrations (0.01-0.1%) were tested to evaluate the sensitivity of the washing steps with regard to this agent (Figure 7). No major differences in the L19-IL2 dose response curves were observed. Conclusively, Tween 20 is an essential component of the washing buffer, but the washing step is very robust with regard to the Tween 20 concentration in the analyzed range.

### Optimization of CTLL-2 concentration and incubation time at 37°C

The CTLL-2 concentration and the incubation time of the test plate at 37°C directly influence the assay outcome and therefore optimization of these parameters is necessary. For this purpose concentrations of approximately 10000, 20000 and 40000 cells per well were seeded in plates that previously had been incubated with L19-IL2 and 7B89biotin. Plates were then incubated at 37°C for about one and two days, respectively, development solution was added and absorbance at 490nm was determined after 4h. Three independent assays were performed and absorbance values corrected by the background signal (0pM L19-IL2) were plotted against the nominal L19-IL2 concentration (Figure 8). As expected higher cell concentrations as well as longer incubation times at 37°C resulted in increased absorbance values (OD490 A/D < B/E < C/F and A/B/C < D/E/F).
To allow better comparability of the results the blank corrected absorbance values were normalized (highest OD490 value was set to 100%, Figure 9) and plotted against the nominal L19-IL2 concentrations. The diagrams illustrate that an increased L19-IL2 concentration is needed to obtain half maximal response, if plates are incubated for two days at 37°C and if more cells are seeded per well, respectively.
In view of a suitable testing conditions two aspects were taken into account, the signal height of control wells (cells incubated in normal growth medium) and the ratio of maximum signal and background (0pM L19-IL2). Absorbance at 490nm of cells incubated in normal growth medium is expected to be higher than absorbance in test wells containing the maximum L19-IL2 concentration. Control values below the maximal L19-IL2 signal may indicate cell death after cell overgrowth due to IL-2 deprivation, which should be avoided. This criteria was fulfilled with all cell concentrations after one day incubation, but only with 10000 seeded cells per well and partially 20000 cells per well after two days incubation at 37°C. A large margin between the L19-IL2 response and the background signal, which is also desirable for valid assay results, was best achieved using a CTLL-2 concentration of 40000 cells/well. Thus, taking into account both parameters one day incubation at 37°C and approximately 40000cells/well were regarded suitable parameters for the assay.

### Short-time stability of L19-IL2

With regard to assay robustness, stability of L19-IL2 at 4°C, room temperature and 37°C was analyzed. In one set of experiments L19-IL2 was diluted and stored overnight at 4°C as well as at room temperature. Then, L19-IL2 dilution series were generated and compared in bioassays to a freshly prepared L19-IL2 standard curve. The response curves of the samples stored at room temperature and 4°C showed both a good congruence with the standard curve. Thus, L19-IL2 shows a good short-term stability at 4°C and room temperature (Figure 10).
Short-term stability at 37°C was also assessed in three experiments. L19-IL2 diluted to 8nM as well as a dilution series were stored overnight at 37°C in a humidified incubator. The next day bioassays were performed using these samples and a freshly prepared L19-IL2 dilution series (set as standard) (Figure 11). Short-term stability was also confirmed at 37°C. Taken together, L19-IL2 is at least over night stable at 4°C, room temperature and 37°C as evaluated by the sandwich IL-2 bioassay. Intra- and interplate variability

To estimate the intra- and interplate variability three assays were performed using three independent dilutions series and three plates per experiment. Figure 12 and Figure 13 show the results of the three preliminary experiments (A/B/C), whereas Figure 12 shows the variability of the dilution series per plate and Figure 13 illustrates interplate variability. As both figures show, some variability was observed in particular in between different plates.
A future purpose for the sandwich IL-2 bioassay, with regard to analytical evaluation of L19-IL2, is to calculate the potency of test samples and to compare them to an L19-IL2 reference standard (i.e. reference of known activity). An initial assessment of the precision of the calculated potency was performed by parallel-line analysis using the PLA software. The parallel-line assay determines the effectiveness of a prepared test sample in comparison to a standard. The standard sample has a defined effectiveness of 1.0. The results of such an analysis is the relative potency of the test sample, which is a dimensionless factor, so it can be given in percent. Its calculation is based on measurable properties that correlate to the logarithm of the concentration. The relative potency can be calculated as the distance of the regression lines at a fixed value of the observable. The implemented mathematical methods in PLA (version 1.2.06) test the measurement values within the linear region for significant slope and non-significant deviation from linearity. Furthermore, the deviation from parallelism of a preparation in comparison to the standard must not be significant. At the end the relative potency of the test sample compared to the standard is reported.
Absorbance values from three experiments with three plates and three dilution series each were used for parallel-line analysis using a significance criterion of 95% for testing linearity, slope and parallelism. Table 1 lists the results of the tests, including slope, linearity and parallelism testing as well as the calculated relative potency of the dilution series 2 and 3. The dilution series 1 was set as standard, resulting in a fixed relative potency of 1.0 and therefore, this series is omitted from the table. Linearity, slope and parallelism testing was successful with exception of assay A, plate 1. Relative potency compared to the standard ranged from 0.740 to 1.322. However, a good accuracy with potency values ranging from 0.920 to 1.064 was achieved, if mean values for each dilution series of one experiment were calculated.

## Claims

1. Method for the determination of the activity of a molecule,
wherein the molecule comprises at least two active parts, and wherein
the first part is capable of modulating the activity of cells, and
the second part can specifically bind to a target,
comprising following steps:
a) incubating the molecule with the target which is covalently or non-covalently bound to a surface and wherein the target is not bound to the surface via cells expressing the target on the cell surface,
b) washing,
c) adding of cells, wherein the activity of the cells is modulated by the molecule
d) determination of the biological effect on these cells,
and wherein in step a) the incubation can be performed by incubating the target and the molecule simultaneously, or by subsequent incubation, for example by first incubating the target, and second incubating the molecule.

2. Method according to claim 1, wherein, the biological effect obtained in step d) is compared to the biological effect obtained with a molecule of known activity.

3. Method for screening for function-inhibiting or neutralizing or blocking components directed against a molecule according to claim 1, comprising following steps:
x) performing a control experiment according to claim 1
xx) performing an experiment according to claim 1, wherein at least one potential function-inhibiting or neutralizing or blocking components is added at any step prior to step c)
xxx) comparing the biological effects obtained in x) and xx)

4. Method according to any of claims 1 to 2, wherein the molecule is an immunotoxin or immunoconjugate.

5. Method according to any of claims 1 to 3, wherein the molecule is a fusion protein.

6. Method according to any of claims 1 to 3 and 5, wherein the first part of the molecule is a cytokine, in particular selected from IL-1, IL-2, IL-12, IL-15, GM-CSF, G-CSF, VEGF, IFNγ, and TNFα.

7. Method according to any of claims 1 to 3, 5, 6, wherein the first part of the molecule is human IL-2.

8. Method according to any of claims 1 to 7, wherein the second part of the molecule is selected from an antibody, antibody fragment or antibody mimetic.

9. Method according to claim 8, wherein the antibody fragment is in scFv, (scFv)2, or small immunoprotein format.

10. Method according to any of the preceding claims, wherein the target specifically bound by the second part of the molecule is ED-B fibronectin or parts thereof comprising the ED-B domain.

11. Method according to claim 8, 9 or 10, wherein the antibody or antibody fragment comprises the CDR regions of L19 and/or comprise at least one Vh and at least one VI chain of the L19-antibody.

12. Method according to claim 7, wherein the cells are selected from CTLL-2, HT-2 and/or TF1β cells.

13. Method according to any of the preceding claims, wherein the biological activity of the cells which is determined, is selected from proliferation, inhibition of cell proliferation, cytotoxicity, viability, cell death, protein biosynthesis and differentiation.

14. Method according to claim 13, wherein proliferation, inhibition of cell proliferation, cytotoxicity, viability, cell death, protein biosynthesis and differentiation is determined colorimetrically, radioactively, fluorometrically and/or by microscopy.

15. Method according to any of the preceding claims, wherein the method fulfils the requirements for quality testing of drugs.

16. Method for the determination of the activity of the L19-IL2 fusion protein, comprising following steps:
a) incubating L19-IL2 with the ED-B fibronectin or parts thereof comprising the ED-B domain which is covalently or non-covalently bound to a surface, wherein the incubation of L19-IL2 and ED-B fibronectin or parts thereof can be performed simultaneously or consecutively,
b) washing
c) adding of IL-2-responsive cells
d) determination of the biological effect on these cells

17. Method according to claim 16, wherein the biological effect is compared to the biological effect of L19-IL2 of known activity.

18. Method according to claim 16 or 17, wherein the cell viability is determined colorimetrically.

19. Method according to any of claims 16 to 18, wherein the target is the ED-B fibronectin fragment 7B89.

20. Kit, comprising
1) a surface covalently or non-covalently coated with a target according to claim 1, and
2) a molecule according to claim 1, which can specifically bind to the target

21. Kit according to claim 20, wherein the surface is a microtitre plate.

22. Kit according to claim 20 or 21, wherein the kit in addition contains cells, wherein the activity of the cells can be modulated by the molecule.

23. Kit according to claim 20, 21 or 22, wherein the kit in addition contains reagents for the determination of the proliferation rate or viability of the cells.

## Amended claims

### Amended claims in accordance with Rule 137(2) EPC.

**1.** Method for the determination of the activity of a molecule,
wherein the molecule comprises at least two active parts, and wherein
the first part is capable of modulating the activity of cells, and
the second part can specifically bind to a target, comprising following steps:
a) incubating the molecule with the target which is covalently or non-covalently bound to a surface and wherein the target is not bound to the surface via cells expressing the target on the cell surface,
b) washing,
c) adding of cells, wherein the activity of the cells is modulated by the molecule
d) determination of the biological effect on these cells,
and wherein in step a) the incubation can be performed by incubating the target and the molecule simultaneously, or by subsequent incubation, for example by first incubating the target, and second incubating the molecule,
wherein
the molecule is a fusion protein, and
the first part of the molecule is a cytokine, in particular selected from IL-1, IL-2,
IL-12, IL-15, GM-CSF, G-CSF, VEGF, IFN_{γ}, and TNFα.

**2.** Method according to claim 1, wherein, the biological effect obtained in step d) is compared to the biological effect obtained with a molecule of known activity.

**3.** Method for screening for function-inhibiting or neutralizing or blocking components directed against a molecule according to claim 1, comprising following steps:
x) performing a control experiment according to claim 1
xx) performing an experiment according to claim 1, wherein at least one potential function-inhibiting or neutralizing or blocking components is added at any step prior to step c)
xxx) comparing the biological effects obtained in x) and xx)

**4.** Method according to any of claims 1 to 3 wherein the first part of the molecule is human IL-2.

**5.** Method according to any of claims 1 to 4, wherein the second part of the molecule is selected from an antibody, antibody fragment or antibody mimetic.

**6.** Method according to claim 5, wherein the antibody fragment is in scFv, (scFv)2, or small immunoprotein format.

**7.** Method according to any of the preceding claims, wherein the target specifically bound by the second part of the molecule is ED-B fibronectin or parts thereof comprising the ED-B domain.

**8.** Method according to claim 5, 6 or 7, wherein the antibody or antibody fragment comprises the CDR regions of L19 and/or comprise at least one Vh and at least one VI chain of the L19-antibody.

**9.** Method according to claim 4, wherein the cells are selected from CTLL-2, HT-2 and/or TF1β cells.

**10.** Method according to any of the preceding claims, wherein the biological activity of the cells which is determined, is selected from proliferation, inhibition of cell proliferation, cytotoxicity, viability, cell death, protein biosynthesis and differentiation.

**11.** Method according to claim 10, wherein proliferation, inhibition of cell proliferation, cytotoxicity, viability, cell death, protein biosynthesis and differentiation is determined colorimetrically, radioactively, fluorometrically and/or by microscopy.

**12.** Method for the determination of the activity of the L19-IL2 fusion protein, comprising following steps:
a) incubating L19-IL2 with the ED-B fibronectin or parts thereof comprising the ED-B domain which is covalently or non-covalently bound to a surface, wherein the incubation of L19-IL2 and ED-B fibronectin or parts thereof can be performed simultaneously or consecutively,
b) washing
c) adding of IL-2-responsive cells
d) determination of the biological effect on these cells

**13.** Method according to claim 12, wherein the biological effect is compared to the biological effect of L19-IL2 of known activity.

**14.** Method according to claim 12 or 13, wherein the cell viability is determined colorimetrically.

**15.** Method according to any of claims 12 to 14, wherein the target is the ED-B fibronectin fragment 7B89.

**16.** Kit, comprising
1) a surface covalently or non-covalently coated with a target according to claim 1, wherein the target is ED-B fibronectin or parts thereof comprising the ED-B domain and
2) a molecule according to claim 1, which can specifically bind to the target, wherein the target specifically bound by the second part of the molecule is ED-B fibronectin or parts thereof comprising the ED-B domain, and
3) cells, wherein the activity of the cells can be modulated by the molecule.

**17.** Kit according to claim 16, wherein the surface is a microtitre plate.

**18.** Kit according to claim 16 or 17, wherein the kit in addition contains reagents for the determination of the proliferation rate or viability of the cells.


SEQUENCE LISTING

<110>  Bayer Schering Pharma Aktiengesellschaft

<120>  Method for determining bioactivity of molecules

<130>  53667AEP

<160>  12

<170>  PatentIn version 3.1

<210>  1
<211>  133
<212>  PRT
<213>  Homo sapiens

<400>  1

<210>  2

<211>  157

<212>  PRT

<213>  Homo sapiens


<400>  2

<210>  3

<211>  116

<212>  PRT

<213>  Artificial Sequence


<220>

<223>  Vh L19

<400>  3

<210>  4

<211>  108

<212>  PRT

<213>  Artificial Sequence

<220>

<223>  Vl L19

<400>  4

<210>  5

<211>  12

<212>  PRT

<213>  Artificial Sequence

<220>

<223>  L-CDR1 L19

<400>  5

Arg Ala Ser Gln Ser Val Ser Ser Ser Phe Leu Ala
1               5                   10

<210>  6

<211>  7

<212>  PRT

<213>  Artificial Sequence

<220>

<223>  L-CDR2 L19

<400>  6

Tyr Ala Ser Ser Arg Ala Thr
1               5

<210>  7

<211>  9

<212>  PRT

<213>  Artificial Sequence

<220>

<223>  L-CDR3 L19

<400>  7

Gln Gln Thr Gly Arg Ile Pro Pro Thr
1               5

<210>  8

<211>  5

<212>  PRT

<213>  Artificial Sequence



<220>

<223>  H-CDR1 L19

<400>  8

Ser Phe Ser Met Ser
1               5

<210>  9

<211>  17

<212>  PRT

<213>  Artificial Sequence



<220>

<223>  H-CDR2 L19

<400>  9

Ser Ile Ser Gly Ser Ser Gly Thr Thr Tyr Tyr Ala Asp Ser Val Lys
1               5                   10                  15

Gly



<210>  10

<211>  7

<212>  PRT

<213>  Artificial Sequence



<220>

<223>  H-CDR3 L19

<400>  10

Pro Phe Pro Tyr Phe Asp Tyr
1               5

<210>  11

<211>  17

<212>  PRT

<213>  Artificial Sequence

<220>

<223>  Fusion linker (e.g. fusion protein L19-IL2)

<400>  11

Glu Phe Ser Ser Ser Ser Gly Ser Ser Ser Ser Gly Ser Ser Ser Ser
1               5                   10                  15

Gly

<210>  12

<211>  12

<212>  PRT

<213>  Artificial Sequence

<220>

<223>  Linker Vh and Vl of L19

<400>  12

Gly Asp Gly Ser Ser Gly Gly Ser Gly Gly Ala Ser
1               5                   10